# EUROPÄISCHE PATENTANMELDUNG

(11) **EP 1 016 383 A1**
(43) Veröffentlichungstag der Anmeldung: **05.07.2000**
(21) Anmeldenummer: 99123715.7
(22) Anmeldetag: 29.11.1999
(51) Int. Cl.: A61B 18/00

(54) **Verfahren und Vorrichtung zum Entfernen von Rauch**

(30) Priorität: 29.12.1998 DE 19860689
(71) Anmelder: Erbe Elektromedizin GmbH., D-72072 Tübingen (DE)
(72) Erfinder: Farin, Günter, 72070 Tübingen (DE)
(74) Vertreter: Bohnenberger, Johannes, Dr.

(57) **Zusammenfassung**

Beim Behandeln von biologischen Geweben in der HF-Chirurgie fallen Kontaminationsstoffe, insbesondere Rauch, an. Es wird vorgeschlagen, eine Absaugeinrichtung (20) derart anzusteuern, daß diese nur dann aktiv wird, wenn an einem Applikator (1) eine zum Zünden eines Lichtbogens hinreichende Spannung ansteht und/oder ein Lichtbogen zwischen dem Applikator (1) und dem Behandlungsbereich brennt, und/oder der Behandlungsbereich eine Temperatur erreicht, bei welcher Rauch entstehen kann.

## Beschreibung

Die Erfindung betrifft ein Verfahren sowie eine Vorrichtung zum Entfernen von Rauch oder dergleichen störenden Kontaminationsstoffen, die bei der Behandlung von biologischem Gewebe in der HF-Chirurgie anfallen, nach dem Oberbegriff des Patentanspruches 1 bzw. 6.

Ein derartiges Verfahren bzw. eine derartige Vorrichtung sind beispielsweise aus den US-Patenten 5,160,334; 5,620,441; 5,108,389; 5,318,516 bekannt. Es wird in diesen Druckschriften vorgeschlagen, bei HF-chirurgischen Operationen oder auch Laseroperationen Rauch oder auch Dampf abzusaugen, um einerseits eine Kontamination umliegender Gewebe zu verhindern, was zu postoperativen Problemen führen kann, und um andererseits ganz allgemein störende Dämpfe zu entfernen, welche die Sicht behindern.

Ein besonderes Problem der bekannten Verfahren bzw. Vorrichtungen besteht darin, daß die dazugehörigen Einrichtungen zur Rauchabsaugung ähnlich wie Staubsauger unangenehme Geräusche erzeugen, welche in Operationsräumen äußerst störend sind. Weiterhin ist es wünschenswert, in Operationsräumen so wenig wie möglich elektrische Geräte laufen zu lassen, die elektrische bzw. elektromagnetische Störungen verursachen.

Der Erfindung liegt die Aufgabe zugrunde, ein Verfahren bzw. eine Vorrichtung der eingangs genannten Art dahingehend aufzuzeigen, daß in einfacher Weise eine Minimierung von Störungen erzielbar ist.

Diese Aufgabe wird verfahrensmäßig durch die Merkmale des Anspruchs 1 und vorrichtungsmäßig durch die Merkmale des Anspruches 6 gelöst.

Ein wesentlicher Punkt der Erfindung liegt darin, daß die Absaugung nur dann einsetzt, wenn tatsächlich störende Kontaminationen (insbesondere Rauch) entstehen können. Damit wird ein zum Stand der Technik vollständig anderer Weg beschritten, da bei allen bekannten Einrichtungen die Rauchabsaugung bereits dann beginnt, wenn eine Aktivierung der Geräte bzw. Präparationsinstrumente eingeleitet wird, also unabhängig davon ist, ob tatsächlich Rauch bzw. Dampf entstehen kann oder nicht.

Es wird also gemäß der vorliegenden Erfindung ein Steuersignal erzeugt, wenn einer (oder mehrere) folgender Betriebszustände vorliegt:
Am Applikator liegt eine zum Zünden eines Lichtbogens hinreichende Spannung an;
Zwischen dem Applikator und dem Gewebe ensteht ein Lichtbogen;
Die Temperatur des Behandlungsbereichs übersteigt einen voreingestellten Temperaturwert, insbesondere 100°C.

Abhängig vom Steuersignal wird die Absaugeinrichtung aktiviert bzw. deaktiviert.

Im ersten Fall, also beim Anlegen einer zum Zünden eines Lichtbogens hinreichenden Spannung wird die Rauchabsaugung sozusagen zum frühestmöglichen Zeitpunkt aktiviert, soweit sicher ist, daß bei Ansetzen des Applikators am Gewebe Rauch/ Dampf entstehen wird.

Bei der zweiten Möglichkeit, also wenn zwischen dem Applikator und dem Gewebe tatsächlich ein Lichtbogen entsteht, wird die Absaugung nur dann eingeschaltet, wenn kontaminierende Stoffe, eben Rauch entstehen. Wenn die zwischen Applikator und Gewebe anstehende HF-Spannung unterhalb dieser kritischen Spannung liegt, kann kein Lichtbogen bzw. kein Funken und damit kein Rauch entstehen, wohl aber Dampf, wenn das Gerät zum Koagulieren bei direktem Kontakt an der Elektrode verwendet wird. In diesem Fall wird aber - wie erwähnt - die Rauchabsaugung nicht eingeschaltet.

Im oben beschriebenen dritten Fall, bei welchem die Absaugung dann eingeschaltet wird, wenn die Temperatur des Behandlungsbereichs einen voreingestellten Temperaturwert überschritten hat, wird Dampf abgesaugt, wenn der Temperaturwert im Bereich von etwa 100°C liegt. Es wird zur Sicherstellung eines Absaugens ausschließlich beim Entstehen von Rauch der Temperaturwert höher gesetzt, also auf über 100°C, da bei niedrigeren Temperaturen Rauch nicht entstehen kann. Durch die oben genannten alternativ bzw. kumulativ anzuwendenden Merkmale wird sichergestellt, daß die Rauchabsaugung so selten wie möglich eingeschaltet wird.

Vorzugsweise wird das die Rauchabsaugung einschaltende Steuersignal dann unterdrückt, wenn sich das HF-chirurgische Gerät in einem Betriebszustand befindet, in welchem kein Rauch entstehen kann, insbesondere dann, wenn dem Behandlungsbereich ein Inertgas oder Edelgas, insbesondere Ar oder He zugeführt wird. In diesem Fall können alle drei obengenannten Kriterien (Zündspannung, Lichtbogen, Temperaturwert) vorliegen, ohne daß Rauch entsteht. Dieser wird nämlich durch die Anwesenheit des Inertgases in seiner Entstehung verhindert, da bei Anwesenheit von Inertgas eine Verbrennung von Gewebe durch die Abwesenheit von Sauerstoff sicher verhindert wird.

Die Absaugeinrichtung wird vorzugsweise hinsichtlich ihrer Absaugleistung derart gesteuert, daß bei einer höheren elektrischen Leistung eine erhöhte Absaugleistung eingestellt wird. Dadurch wird sichergestellt, daß der Geräusch- bzw. Störungspegel der Absaugeinrichtung so niedrig wie möglich gehalten wird, dabei aber gleichzeitig eine hinreichende Absaugwirkung einstellbar ist.

Das Steuersignal wird vorzugsweise nach Beendigung des ersten oder zweiten Betriebszustandes (Temperatur, Lichtbogen) für eine vorbestimmte Zeitdauer aufrechterhalten, um sicherzustellen, daß entstandener Rauch oder Dampf vollständig abgesaugt wird.

Bei einer weiteren bevorzugten Ausführungsform wird die Absaugeinrichtung bei Vorliegen einer Lichtbogen-Zündspannung zwischen dem Applikator und dem Gewebe auf eine niedrigere Anlaufleistung gebracht, bis entweder ein Lichtbogen oder eine erhöhte Temperatur festgestellt wird, die dann wiederum dazu führen, daß die Absaugleistung erhöht wird. Dadurch wird sichergestellt, daß dann, wenn der Operateur die Betätigungseinrichtung (Pedal, Knopf am Applikator) betätigt, um Gewebe zu schneiden oder zu koagulieren, das Absauggerät schon in einen aktivierten Zustand überführt wird, um bei tatsächlichem Auftreten von Rauch oder Dampf sofort diesen mit erhöhter Absaugleistung, die vom Anlaufbetriebszustand aus leichter bzw. schneller zu erreichen ist, als vom völlig deaktivierten Zustand, absaugen zu können.

Zur Durchführung dieses Verfahrens sind folgende Einrichtungen vorgesehen:

Eine Abtasteinrichtung zum Abtasten einer am Applikator anliegenden Spannung, eine erste Vergleichereinrichtung in der Steuereinrichtung zum Vergleichen der Spannung mit einem voreinstellbaren Zündspannungswert und zum Abgeben eines ersten Steuersignals zur Aktivierung der Absaugeinrichtung dann, wenn die Spannung den Zündspannungswert mindestens erreicht;
eine zweite Abtasteinrichtung zum Abtasten eines Betriebszustandes des HF-chirurgischen Geräts und zum Abgeben eines Betriebszustandsignals, eine Erkennungseinrichtung in der Steuereinrichtung zum Abtasten des Betriebszustandsignals und zum Abgeben eines zweiten Steuersignais zur Aktivierung der Absaugeinrichtung dann, wenn aufgrund des Betriebszustandsignals das Vorliegen eines Lichtbogens zwischen dem Behandlungsbereich und dem Applikator erkannt wird;
eine dritte Abtasteinrichtung zum Abtasten einer Temperatur des Behandlungsbereichs oder zum Abtasten elektromagnetischer Strahlung, insbesondere Lichtwellen, die nahe oder bei dem Behandlungsbereich entstehen, eine zweite Vergleichereinrichtung in der Steuereinrichtung zum Vergleichen der abgetasteten Temperatur oder Strahlung mit einem voreinstellbaren Temperaturwert oder Strahlungswert und zum Abgeben eines dritten Steuerungssignals zum Aktivieren der Absaugeinrichtung dann, wenn die abgetastete Temperatur (Strahlung) den voreinstellbaren Wert überschreitet.

Die zweite Abtasteinrichtung kann im wesentlichen so ausgebildet sein, wie dies beispielsweise in der DE 25 04 280 oder der EP 0 219 568 beschrieben ist, auf die hier ausdrücklich Bezug genommen wird.

Die dritte Abtasteinrichtung stellt vorzugsweise eine Strahlungsemission fest, die sowohl Infrarotstrahlung als auch sichtbares Licht umfassen kann. Man kann also mit dieser dritten Abtasteinrichtung feststellen, ob der Behandlungsbereich eine Temperatur aufweist, bei welcher Dampf bzw. Rauch entstehen kann (Abtastung von Wärmestrahlung), oder ob ein Lichtbogen entsteht. Im letzteren Fall ist es notwendig, einen relativ großen Winkel zu erfassen, da der exakte Ort, zu welchem der Lichtbogen wandert, meist nicht eindeutig festlegbar ist.

Vorzugsweise ist eine Betriebszustandserkennungseinrichtung vorgesehen, um einen Betriebszustand des HF-chirurgischen Geräts zu erkennen, in dem es sich gerade befindet. In Abhängigkeit von diesem Betriebszustand würde dann entweder das Steuersignal zur Ansteuerung der Absaugeinrichtung "durchgeschaltet" oder aber unterdrückt. Es wird insbesondere dann unterdrückt, wenn der Betriebszustand derart eingestellt wurde, daß kein Rauch oder die Sicht behindernder Dampf entstehen kann. Dies ist insbesondere dann der Fall, wenn das HF-chirurgische Gerät dem Behandlungsbereich ein Edelgas (He, Ar) zuführt, so daß der Lichtbogen im Edelgas brennt und der Behandlungsbereich frei von Sauerstoff gehalten wird.

Weiterhin ist vorzugsweise eine Leistungsabtasteinrichtung vorgesehen, welche die elektrische Leistung feststellt, die das HF-chirurgische Gerät dem Behandlungsbereich zuführt. In Abhängigkeit von dieser Leistung wird ein Einstellsignal erzeugt, das die Absaugleistung der Absaugeinrichtung derart steuert, daß bei höherer elektrischer Leistung keine höhere Absaugleistung eingestellt wird als bei einer niedrigeren elektrischen Leistung. Dadurch kann gewährleistet werden, daß bei nur geringer elektrischer Leistung und demzufolge auch nur geringerer Rauchentwicklung eine geringere Absaugleistung und damit geringere Störungen eingestellt werden.

Vorzugsweise ist eine Verzögerungseinrichtung vorgesehen, die das Abschalten der Absaugeinrichtung über einen vorbestimmten Zeitraum hinweg verzögert, wenn das zweite oder das dritte Steuersignal abgeschaltet werden, also nachdem ein Lichtbogen erloschen oder die Temperatur des Behandlungsbereiches (Strahlung des Behandlungsbereiches) unter einen vorbestimmten Pegel abgefallen sind. Dadurch wird gewährleistet, daß Reste von Rauch auch noch abgesaugt werden und so keine Kontamination des Behandlungsbereiches erfolgen kann. In diesem Fall ist die Verzögerungseinrichtung vorzugsweise derart einstellbar hinsichtlich der von ihr erzeugten Abschaltverzögerung einstellbar, daß bei einer steigenden elektrischen Leistung bzw. steigender Zeitdauer, während derer eine vorbestimmte Mindestleistung überschritten wird (bzw. ein Lichtbogen brennt) diese Verzögerungszeit eingestellt wird. Dadurch wird ebenfalls gewährleistet, daß erhöhte Rauchmengen sicher abgesaugt werden.

Um sicherzustellen, daß möglichst sofort nach Beginn der Dampf- oder Rauchentwicklung die Absaugung effizient arbeitet, wird weiterhin eine Anlaufsteuereinrichtung vorgesehen, die auf das erste Steuersignal hin die Absaugeinrichtung auf eine niedrigere Anlaufleistung und bei Vorliegen des zweiten oder dritten Steuersignais hin auf eine höhere Absaugleistung einstellt. Es läuft also die Absaugeinrichtung bereits dann los, wenn eine an sich zündfähige Spannung vorliegt, was sofort bei Betätigung des Gerätes (Fingerschalter, Fußschalter) der Fall ist. Von dieser niedrigeren Anlaufleistung aus kann dann sehr schnell auf eine höhere Absaugleistung übergegangen werden.

Besonders vorteilhaft ist die Verwendung der Erfindung in Zusammenhang mit HF-Chirurgie im endoskopischen Anwendungsbereich. In diesem Fall nämlich, wenn also der Rauch in einer Körperhöhle entsteht, ist die Kontaminationsgefahr besonders hoch. Darüber hinaus ist auch in diesem Fall der Rauch besonders störend, so daß eine effiziente Rauchabsaugung besonders vorteilhaft ist. Schließlich ist auch noch zu erwähnen, daß Rauch (auch Dampf) die Optik des Endoskops verschmutzen und damit die Operation schwierig machen kann. Überraschenderweise hat es sich gezeigt, daß gerade bei endoskopischen Operationen die Rauchabsaugung besonders wünschenswert ist.

Nachfolgend wird die Erfindung anhand eines Ausführungsbeispiels in Zusammenhang mit einer Zeichnung näher erläutert, welche in stark schematisierter Ansicht ein HF-chirurgisches Gerät mit Steuerungseinrichtung zeigt.

In dem Ausführungsbeispiel ist ein HF-chirurgisches Gerät mit der Bezugsziffer 10 bezeichnet, das einen Hochfrequenz-Leistungsgenerator 11 und Einstellelemente 12 aufweist, über welche die Betriebsarten des Gerätes, z. B. die Betriebsarten "Schneiden", "Koagulieren", "APC" (Argon-Plasma-Koagulation) usw. einstellbar sind. Das HF-chirurgische Gerät 10 ist in üblicher Weise mit einem Applikator 1 verbunden, über welche die HF-Leistung dem Behandlungsbereich eines biologischen Gewebes zuführbar ist. Vorzugsweise ist der Applikator 1 so ausgestaltet, daß er für endoskopische Operationen verwendbar ist, also durch den Arbeitskanal eines Endoskops hindurchführbar ist.

Weiterhin ist eine Betätigungseinrichtung 2 vorgesehen, über welche das HF-chirurgische Gerät vom Operateur angesteuert werden kann. Im Ausführungsbeispiel ist diese Betätigungseinrichtung 2 als Bedien-Taste ausgebildet, die am Applikator 1 angebracht ist. Bei einem Applikator für endoskopische Operationen ist diese Betätigungseinrichtung beispielsweise als Pedal ausgeführt.

Zum Absaugen von Dampf oder Rauch ist eine Absaugeinrichtung 20 vorgesehen, die über eine Saugleitung 22 mit einer Düse 21 verbunden ist, welche am Applikator 1 angebracht ist. Die Düse 21 sitzt hierbei derart, daß ihre Mündung möglichst nahe an dem Ort liegt, an welchem die störenden Kontaminationsstoffe abgesaugt werden sollen.

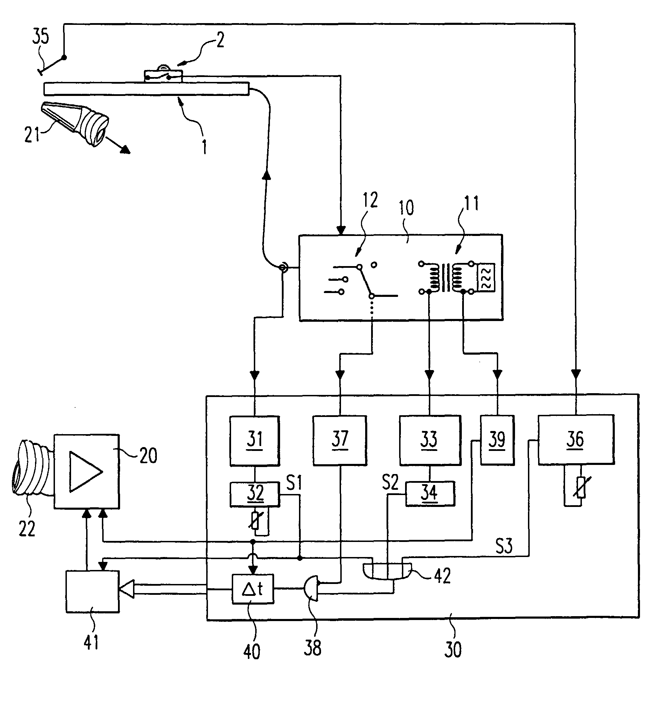

Zur Ansteuerung der Absaugeinrichtung 20 ist eine Steuereinrichtung 30 vorgesehen. Die Steuereinrichtung 30 umfaßt eine Zündspannungsabtasteinrichtung 31, welche die am Applikator 1 anliegende bzw. von dem HF-chirurgischen Gerät 10 erzeugte Spannung abtastet. Der Ort, an welchem die Spannung abgetastet wird, ist in der Zeichnung schematisiert mit der Ausgangsleitung vom HF-chirurgischen Gerät zum Applikator 1 angegeben, jedoch wird dies in der Praxis immer ein zur tatsächlichen Zündspannung proportionales Signal sein, das vom HF-chirurgischen Gerät 10 erzeugt wird. Die von der Zündspannungsabtasteinrichtung 31 in der Steuereinrichtung 30 abgetastete Spannung wird mittels einer ersten Vergleichereinrichtung 32 mit einem voreinstellbaren Spannungswert verglichen, welcher einer Zündspannung entspricht, die insbesondere beim Schneiden zum Zünden eines Funkens bzw. Lichtbogens notwendig ist. Sobald diese Spannung somit erkannt wird, besteht die Möglichkeit, daß tatsächlich ein Schneidvorgang eingeleitet wird, bei welchem Rauch entstehen kann. Bei niedrigeren Spannungen kann kein Rauch entstehen, sondern höchstens (Wasser-) Dampf. Von der ersten Vergleichereinrichtung 32 wird ein Steuersignal S1 erzeugt.

Weiterhin ist eine Betriebszustandsabtasteinrichtung 33 vorgesehen, welche den Betriebszustand des HF-chirurgischen Gerätes 10 abtastet und das mit einer Erkennungseinrichtung 34 verbunden ist, die derart ausgebildet ist, daß dann ein zweites Steuersignal S2 abgegeben wird, wenn die Erkennungseinrichtung 34 aus dem Ausgangssignal der Betriebszustandsabtasteinrichtung 33 erkennt, daß ein Lichtbogen zwischen dem Applikator 1 und dem Behandlungsbereich brennt. Eine derartige Erkennungseinrichtung ist beispielsweise in der DE 25 04 280 oder in der EP 0 219 568 beschrieben.

Weiterhin ist eine Temperaturabtasteinrichtung 35 am Applikator 1 vorgesehen, welche z. B. als Pyrometer oder auch als Empfänger für sichtbares Licht ausgebildet sein kann, um die Temperatur des Behandlungsbereiches oder Lichtemissionen in diesem Bereich (z. B. bei Entstehung eines Funkens) abzutasten. Das Ausgangssignal der Temperaturabtasteinrichtung 35 wird einer zweiten Vergleichereinrichtung 36 zugeführt, in der es mit einem voreinstellbaren Wert verglichen werden kann, so daß bei Überschreiten dieses voreingestellten Wertes ein drittes Steuersignal S3 erzeugt wird, und zwar dann, wenn die Temperatur des Behandlungsbereiches so hoch ist, daß Rauch entstehen kann.

In der Steuereinrichtung 30 ist weiterhin eine Betriebszustandserkennungseinrichtung 37 vorgesehen, welche den Betriebszustand des HF-chirurgischen Gerätes insofern abtastet, als sie die Stellung der Einstellelemente 12 abtastet. Dann, wenn die Betriebszustandserkennungseinrichtung 37 feststellt, daß das HF-chirurgische Gerät 10 sich in einem Betriebszustand befindet, in welchem keine kontaminierenden Stoffe (insbesondere Rauch) entstehen, z. B. in der Betriebsart "APC", sendet sie ein Unterdrückungssignal zu einer Unterdrückungseinrichtung 38, die im vorliegenden Fall als UND-Gatter mit einem invertierenden Eingang ausgebildet ist.

Die Steuersignale S1, S2 und S3 werden einem Oder-Gatter 42 zugeführt, dessen Ausgang auf dem nicht-invertierenden Eingang des UND-Gatters 38 liegt. Der Ausgang des UND-Gatters führt auf den Eingang einer Verzögerungseinrichtung 40, welche ein Steuersignal über eine Anlaufsteuerung 41 an die Absaugeinrichtung 20 zu deren Ansteuerung gibt. Bei dieser Ausführungsform der Erfindung wird also immer dann, wenn eines der Steuersignal S1-S3 vorliegt und der Betriebszustand des HF-chirurgischen Gerätes derart ist, daß kontaminierende Stoffe entstehen können, die Absaugeinrichtung 20 eingeschaltet. Wenn die drei Steuersignale S1, S2 oder S3 nicht mehr vorliegen, bewirkt die Verzögerungseinrichtung 40 ein verzögertes Abschalten der Absaugeinrichtung 20, so daß sichergestellt wird, daß Reste der kontaminierenden Stoffe im Bereich zwischen dem biologischen Gewebe und dem Applikator 1 abgesaugt werden.

Weiterhin ist eine Leistungsabtasteinrichtung 39 vorgesehen, welche ein der vom HF-chirurgischen Gerät 10 erzeugten wirksamen Leistung entsprechendes Signal erzeugt, das zum einen der Verzögerungseinrichtung 40 und zum anderen der Absaugeinrichtung 20 zugeführt wird. Mit diesem leistungsabhängigen Signal wird in der Verzögerungseinrichtung 40 die Nachlaufzeit so erhöht oder verringert, daß bei höherer Leistungsabgabe durch das HF-chirurgische Gerät eine längere Nachlaufzeit eingestellt wird als bei einer geringeren Leistung. Dadurch wird sichergestellt, daß bei erhöhter Erzeugung von kontaminierenden Stoffen, insbesondere von Rauch, auch nach Beendigung eines Schneidvorganges der entstandene Rauch abgesaugt wird. Zusätzlich (oder auch alternativ) kann die Zeit mit in die Verlängerung der Nachlaufdauer einfließen, über welche überhaupt eine Leistung erzeugt und damit dem Gewebe zugeführt wurde. Es wird dann nicht der Leistung entsprechend sondern der vom HF-chirurgischen Gerät erzeugten Energie (Arbeit) entsprechend verlängert abgesaugt.

Das der Leistung entsprechende Signal wird darüber hinaus direkt der Absaugeinrichtung 20 zugeführt, so daß diese bei höherer (Schneid-) Leistung auch mit einer höheren Absaugleistung arbeitet als bei niedrigerer HF-Leistung.

Aus obigem geht hervor, daß ein wesentlicher Punkt der Erfindung darin liegt, daß die Absaugeinrichtung in Abhängigkeit von den Parametern der vom HF-chirurgischen Gerät erzeugten Energie betätigt wird, welche im wesentlichen unmittelbar für die Erzeugung von kontaminierenden Stoffen, insbesondere von Rauch oder aber auch von Dampf verantwortlich sind. Auf diese Weise wird eine Minimierung des Absaugvorganges mit den damit verbundenen Storungen bei gleichzeitiger Beibehaltung oder sogar Steigerung der Absaugeffizienz gewährleistet.

### Bezugszeichenliste

- 1: Applikator
- 2: Betätigungseinrichtung
- 10: HF-chirurgisches Gerät
- 11: Leistungsgenerator
- 12: Einstellelemente
- 20: Absaugeinrichtung
- 21: Düse
- 22: Saugleitung
- 30: Steuereinrichtung
- 31: Zündspannungsabtasteinrichtung
- 32: erste Vergleichereinrichtung
- 33: Betriebszustandsabtasteinrichtung
- 34: Erkennungseinrichtung
- 35: Temperaturabtasteinrichtung
- 36: zweite Vergleichereinrichtung
- 37: Betriebszustandserkennungseinrichtung
- 38: Unterdrückungseinrichtung
- 39: Leistungsabtasteinrichtung
- 40: Verzögerungseinrichtung
- 41: Anlaufsteuerungseinrichtung
- 42: Oder-Gatter
- S1: Zündspannungssteuersignal
- S2: Lichtbogensteuersignal
- S3: Temperatursteuerungssignal

## Patentansprüche

1. Verfahren zum Entfernen von Rauch oder dergleichen störenden Kontaminationsstoffen, die bei der Behandlung von biologischem Gewebe in der HF-Chirurgie anfallen,
- mit einem HF-chirurgischen Gerät, das eine elektrische Leistung auf einen Behandlungsbereich des Gewebes mittels eines Applikators überträgt;
- mit einer steuerbaren Absaugeinrichtung oder dergleichen Gerät zum Entfernen von Kontaminationsstoffen im Umfeld des Applikators und
- mit einer Steuereinrichtung zum Steuern der Absaugeinrichtung in Abhängigkeit von einem Betriebszustand des HF-chirurgischen Geräts,
**gekennzeichnet**
durch die Verfahrensschritte
- es wird ein Steuersignal erzeugt, wenn folgende Betriebszustände vorliegen:
a) am Applikator liegt eine zum Zünden eines Lichtbogens hinreichende Spannung an und/oder
b) zwischen dem Applikator und dem Gewebe entsteht ein Lichtbogen und/oder
c) eine Temperatur des Behandlungsbereichs übersteigt einen voreingestellten Temperaturwert, insbesondere 100° Celsius;
- bei einem Anschalten des Steuersignals wird die Absaugeinrichtung aktiviert, bei einem Abschalten des Steuersignals deaktiviert.

2. Verfahren nach Anspruch 1,
**dadurch gekennzeichnet,**
daß das Steuersignal dann unterdrückt wird, wenn sich das HF-chirurgische Gerät in einem Betriebszustand befindet, in welchem kein Rauch entstehen kann, insbesondere dann, wenn dem Behandlungsbereich ein Inertgas, insbesondere Ar oder He zugeführt wird.

3. Verfahren nach einem der vorhergehenden Ansprüche,
**dadurch gekennzeichnet,**
daß die Absaugeinrichtung hinsichtlich ihrer Absaugleistung derart gesteuert wird, daß bei einer höheren elektrischen Leistung eine erhöhte Absaugleistung eingestellt wird.

4. Verfahren nach einem der vorhergehenden Ansprüche,
**dadurch gekennzeichnet,**
daß das Steuersignal nach Beendigung eines der Betriebszustände b) oder c) für eine vorbestimmte Zeitdauer aufrechterhalten wird.

5. Verfahren nach Anspruch 3,
**dadurch gekennzeichnet,**
daß die Absaugeinrichtung bei Vorliegen des Betriebszustands a) und Nicht-Vorliegen eines der Betriebszustände b) oder c) auf eine niedrigere Anlaufleistung und bei einem darauffolgenden Übergang in den Betriebszustand b) oder c) auf eine erhöhte Absaugleistung eingestellt wird.

6. Vorrichtung zum Entfernen von Rauch oder dergleichen störenden Kontaminationsstoffen, die bei der Behandlung von biologischem Gewebe in der HF-Chirurgie anfallen, insbesondere zur Durchführung des Verfahrens nach Anspruch 1,
- mit einem HF-chirurgischen Gerät (10), das eine elektrische Leistung auf einen Behandlungsbereich des Gewebes mittels eines Applikators (1) überträgt,
- mit einer steuerbaren Absaugeinrichtung (20) oder dergleichen Gerät zum Entfernen von Kontaminationsstoffen im Umfeld des Applikators (1) und
- mit einer Steuereinrichtung (30) zum Steuern der Absaugeinrichtung (20) in Abhängigkeit von einem Betriebszustand des HF-chirurgischen Geräts (10),
**gekennzeichnet**
durch
eine erste Abtasteinrichtung (31) zum Abtasten einer am Applikator anliegenden Spannung;
eine erste Vergleichereinrichtung (32) in der Steuereinrichtung (30) zum Vergleichen der Spannung mit einem voreinstellbaren Zündspannungswert und zum Abgeben eines ersten Steuersignals (S1) zur Aktivierung der Absaugeinrichtung, dann, wenn die Spannung den Zündspannungswert mindestens erreicht, und/oder
eine zweite Abtasteinrichtung (33) zum Abtasten eines Betriebszustands des HF-chirurgischen Geräts (10) und zum Abgeben eines Betriebszustandssignals;
eine Erkennungseinrichtung (34) in der Steuereinrichtung (30) zum Abtasten des Betriebszustandssignals und zum Abgeben eines zweiten Steuersignals (S2) zur Aktivierung der Absaugeinrichtung (20) dann, wenn aufgrund des Betriebszustandssignals das Vorliegen eines Lichtbogens zwischen dem Behandlungsbereich und dem Applikator (1) erkannt wird, und/oder
eine dritte Abtasteinrichtung (35) zum Abtasten einer Temperatur des Behandlungsbereichs oder elektromagnetischer Strahlung, insbesondere Lichtwellen, die nahe oder bei dem Behandlungsbereich entstehen;
eine zweite Vergleichereinrichtung (36) in der Steuereinrichtung (30) zum Vergleichen der abgetasteten Temperatur oder Strahlung mit einem voreinstellbaren Temperaturwert oder Strahlungswert und zum Abgeben eines dritten Steuersignals (S3) zum Aktivieren der Absaugeinrichtung (20) dann, wenn die abgetastete Temperatur den voreinstellbaren Temperaturwert überschreitet.

7. Vorrichtung nach Anspruch 6,
**gekennzeichnet**
durch eine Betriebszustandserkennungseinrichtung (37) zum Erkennen eines Betriebszustands des HF-chirurgischen Geräts (10) und zum Erzeugen eines, das Steuersignal mittels einer Unterdrückungseinrichtung (38) unterdrückenden Steuerungssignals dann, wenn sich das HF-chirurgische Gerät (10) in einem Betriebszustand befindet, in welchem kein Rauch entstehen kann, insbesondere dann, wenn dem Behandlungsbereich ein Inertgas, insbesondere Ar oder He zugeführt wird.

8. Vorrichtung nach Anspruch 6,
**gekennzeichnet**
durch eine Leistungsabtasteinrichtung (39) zum Abtasten der elektrischen Leistung und zum Abgeben eines Einstellsignals zum Steuern einer Absaugleistung der Absaugeinrichtung (20) derart, daß bei einer höheren elektrischen Leistung eine höhere Absaugleistung eingestellt wird als bei niedrigerer elektrischer Leistung.

9. Vorrichtung nach Anspruch 6,
**gekennzeichnet**
durch eine Verzögerungseinrichtung (40) zum Verzögern eines Abschaltens der Absaugeinrichtung (20) über einen vorbestimmten Zeitraum, wenn das zweite oder das dritte Steuersignal (S2, S3) abgeschaltet werden.

10. Vorrichtung nach Anspruch 9,
**dadurch gekennzeichnet,**
daß die Verzögerungseinrichtung (40) eine Einstelleinrichtung umfaßt, welche den vorbestimmten Zeitraum mit steigender elektrischer Leistung und/oder steigender Zeitdauer, während derer eine vorbestimmte Mindestleistung überschritten wird, erhöht.

11. Vorrichtung nach Anspruch 6,
**gekennzeichnet**
durch eine Anlaufsteuereinrichtung (41), die auf das erste Steuersignal (S1) in die Absaugeinrichtung (20) auf eine niedrigere Anlaufleistung und bei Vorliegen des zweiten oder dritten Steuersignals (S2, S3) auf eine höhere Absaugleistung einstellt.

12. Verwendung der Vorrichtung nach einem der Ansprüche 6-11 zur Absaugung von Rauch bei einem Applikator für endoskopische Behandlungen.
